# EUROPEAN PATENT APPLICATION

(11) **EP 4 197 589 A2**
(43) Date of publication of application: **21.06.2023**
(21) Application number: 22214045.1
(22) Date of filing: 16.12.2022
(51) Int. Cl.: A61N 1/375

(54) **BIOSTIMULATOR HAVING PACING ELEMENT FOR DEEP SEPTAL PACING**

(30) Priority: 17.12.2021 US 202163291261 P
(71) Applicant: Pacesetter, Inc., Sylmar, CA 91342 (US)
(72) Inventor: BORZIN, Gene A, Sylmar, 91342 (US); SOMOGYI, Zoltan, Sylmar, 91342 (US); RYU, Kyungmoo, Sylmar, 91342 (US); NIX, Kyle J, Sylmar, 91342 (US); VICTORINE, Keith, Sylmar, 91342 (US)
(74) Representative: Barker Brettell Sweden AB

(57) **Abstract**

A biostimulator (100) and a pace mapping system (1500) for deep septal pacing. The biostimulator (100) includes a pacing element (108) that extends distally beyond a fixation element (!06). The pacing element (108) is insulated between a distal tip (212) of the fixation element (106) and a distal portion (404) of the pacing element (108). The pace mapping system (1500) can be used to determine a distance between a septal wall (110) of a heart septum (104) and a bundle branch (112) within the septum (104). The pacing element (108) can be selected based on the distance, and delivered into the septum (104) to pace the bundle branch (112). Other embodiments are also described and claimed.

## Description

### TECHNICAL FIELD

The present disclosure relates to biostimulators and related mapping and transport systems. More specifically, the present disclosure relates to leadless biostimulators and related mapping and transport systems useful for deep septal pacing.

### BACKGROUND

Cardiac pacing by an artificial pacemaker provides an electrical stimulation of the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death.

Leadless cardiac pacemakers incorporate electronic circuitry at the pacing site and eliminate leads, thereby avoiding shortcomings associated with conventional cardiac pacing systems. Leadless cardiac pacemakers can be anchored at the pacing site, e.g., in a right ventricle and, for dual-chamber pacing, in a right atrium, by an anchor. A delivery system can be used to deliver the leadless cardiac pacemakers to the target anatomy.

Cardiac pacing of the His-bundle is clinically effective and advantageous by providing a narrow QRS affecting synchronous contraction of the ventricles. His-bundle pacing in or near a membranous septum of a heart, however, has some drawbacks. The procedure is often long in duration and requires significant fluoroscopic exposure. Furthermore, successful His-bundle pacing cannot always be achieved. Pacing thresholds are often high, sensing is challenging, and success rates can be low.

Deep septal pacing is an alternative to His-bundle pacing. Deep septal pacing involves pacing past the His-bundle toward the right ventricle apex. More particularly, deep septal pacing targets the left bundle branch below the His site. Deep septal pacing has been achieved using a lead in which the electrode penetrates several millimeters into the septum. Pacing thresholds associated with deep septal pacing are potentially lower than with His-bundle pacing, and clinical efficacy of the approach has been demonstrated.

### SUMMARY

Existing leads are not well suited to pacing the deep septal area. The leads lack the ability to control penetration into the target tissue, and thus, the leads may not penetrate beyond the fibrous endocardial tissue. Furthermore, the leads suffer from the shortcomings associated with conventional cardiac pacing systems. Accordingly, there is a need for a leadless biostimulator and systems for delivering a pacing element of the leadless biostimulator to the deep septal area for deep septal pacing of a heart.

The present invention is defined in the independent claims. Further embodiments of the invention are defined in the dependent claims.

A biostimulator is described. In an embodiment, the biostimulator includes a housing having a longitudinal axis. The housing contains an electronics compartment. A fixation element is connected to the housing. The fixation element includes an outer helix extending about the longitudinal axis to a first distal tip. A pacing element is connected to the housing. The pacing element extends along the longitudinal axis to a second distal tip. The second distal tip of the pacing element is distal to the first distal tip of the fixation element. A pacing insulation covers an outer surface of the pacing element between the first distal tip and a distal portion of the pacing element having the second distal tip.

A pacing element is described. In an embodiment, the pacing element includes a body, a proximal threaded portion, and a distal portion. The distal portion has a distal tip. The body extends from the proximal threaded portion to the distal portion. A pacing insulation is on an outer surface of the body between the proximal threaded portion and the distal portion.

A pace mapping system is described. In an embodiment, the pace mapping system includes a steerable sheath having a central lumen. The pace mapping system includes a pace mapping tool including an elongated body. The elongated body has a distal body end. The pace mapping system includes a sensing tip connected to the distal body end. The sensing tip is configured to pierce a target tissue.

The above summary does not include an exhaustive list of all aspects of the present invention. It is contemplated that the invention includes all systems and devices that can be practiced from all suitable combinations of the various aspects summarized above, as well as those disclosed in the Detailed Description below and particularly pointed out in the claims filed with the application. Such combinations have particular advantages not specifically recited in the above summary.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings.
FIG. 1 is a diagrammatic cross section of a patient heart illustrating an example implantation of a biostimulator in a target anatomy, in accordance with an embodiment.
FIG. 2 is a side view of a biostimulator having a pacing element, in accordance with an embodiment.
FIG. 3 is an end view of a biostimulator having a pacing element, in accordance with an embodiment.
FIG. 4 is a side view of a biostimulator having a helical electrode, in accordance with an embodiment.
FIG. 5 is a side view of a distal portion of a biostimulator having a helical electrode, in accordance with an embodiment.
FIG. 6 is a side view of a biostimulator having an insulating sleeve, in accordance with an embodiment.
FIG. 7 is a side view of a distal portion of a biostimulator having an insulating sleeve, in accordance with an embodiment.
FIG. 8 is a side view of a biostimulator having a conical electrode, in accordance with an embodiment.
FIG. 9 is a side view of a distal portion of a biostimulator having a conical electrode, in accordance with an embodiment.
FIG. 10 is a cross-sectional view of a distal portion of a biostimulator having a pacing element, in accordance with an embodiment.
FIG. 11 is a pictorial view of a biostimulator configured to receive one or more interchangeable pacing elements, in accordance with an embodiment.
FIG. 12 is a side view of a pacing element having an internal torque feature, in accordance with an embodiment.
FIG. 13 is a side view of a pacing element having an external torque feature, in accordance with an embodiment.
FIG. 14 is a side view of a biostimulator having an outer insulation, in accordance with an embodiment.
FIG. 15 is a side view of a pace mapping system, in accordance with an embodiment.
FIG. 16 is a flowchart of a method of stimulating a bundle branch using a biostimulator having a pacing element, in accordance with an embodiment.
FIGS. 17-18 are pictorial views of operations of a method of stimulating a bundle branch using a biostimulator having a pacing element, in accordance with an embodiment.

### DETAILED DESCRIPTION

Embodiments describe a biostimulator, such as a leadless pacemaker, having a pacing element that extends distal to a fixation element. As described below, the biostimulator can be used to perform deep septal pacing of a heart. The biostimulator may, however, be used in other applications, such as deep brain stimulation. Thus, reference to the biostimulator as being a cardiac pacemaker for deep septal pacing is not limiting.

In various embodiments, description is made with reference to the figures. However, certain embodiments may be practiced without one or more of these specific details, or in combination with other known methods and configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description. Reference throughout this specification to "one embodiment," "an embodiment," or the like, means that a particular feature, structure, configuration, or characteristic described is included in at least one embodiment. Thus, the appearance of the phrase "one embodiment," "an embodiment," or the like, in various places throughout this specification are not necessarily referring to the same embodiment. Furthermore, the particular features, structures, configurations, or characteristics may be combined in any suitable manner in one or more embodiments.

The use of relative terms throughout the description may denote a relative position or direction. For example, "distal" may indicate a first direction along a longitudinal axis of a biostimulator. Similarly, "proximal" may indicate a second direction opposite to the first direction. Such terms are provided to establish relative frames of reference, however, and are not intended to limit the use or orientation of a biostimulator to a specific configuration described in the various embodiments below.

In an aspect, a biostimulator includes a pacing electrode that extends distally beyond a fixation element. For example, a distal tip of the pacing element can be 5-20 mm, e.g., 7 mm, 12 mm, 15 mm, or 17 mm, distal to a distal tip of the fixation element. Accordingly, when the fixation element is anchored in a septal wall of a heart, the pacing element can extend into the septal wall to pace a bundle branch in the heart septum. The fixation element may also be used for pacing. For example, the fixation element can be electrically connected to a housing of the biostimulator that acts as an anode to provide anodic pacing to the septal wall or a bundle branch within the septal wall. Alternatively, the fixation element may be electrically connected to an electrical feedthrough of the biostimulator that delivers pacing pulses to the fixation element and the pacing element for anodic or cathodic pacing of the septal wall or bundle branch(es) within the septal wall. The pacing element may be interchangeable, and thus, an appropriately-sized pacing element may be selected and installed in the biostimulator based on a depth of the target bundle branch within the septal wall. A pace mapping system is described that can determine the depth of the target bundle branch (to facilitate pacing element selection). The pace mapping system may be used to guide a transport system carrying the biostimulator to a pacing site at the septal wall.

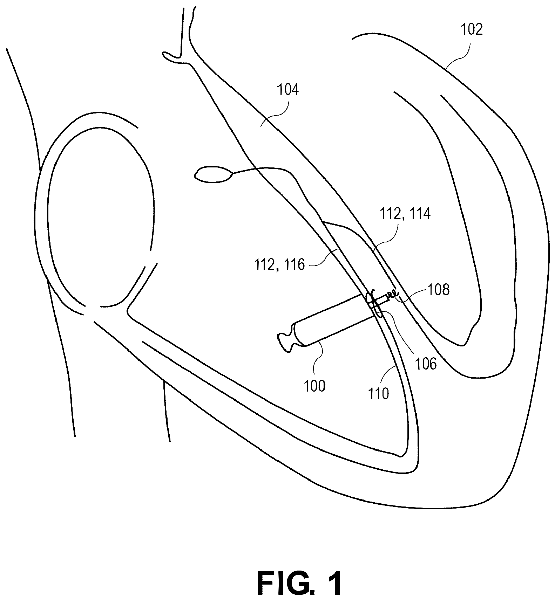

Referring to FIG. 1, a diagrammatic cross section of a patient heart illustrating an example implantation of a biostimulator in a target anatomy is shown in accordance with an embodiment. A leadless biostimulator system, e.g., a cardiac pacing system, includes one or more biostimulators 100. The biostimulators 100 can be implanted in a patient heart 102, and can be leadless (and thus, may be leadless cardiac pacemakers). Each biostimulator 100 can be placed in a cardiac chamber, such as a right atrium and/or right ventricle of the heart 102, or attached to an inside or outside of the cardiac chamber. For example, the biostimulator 100 can be attached to a septum 104 of the heart 102. More particularly, the biostimulator 100 can be delivered to the septum 104, and one or more elements, such as a fixation element 106 and/or a pacing element 108 can pierce a septal wall 110 of the septum 104 to engage and anchor the biostimulator 100 to the tissue. In a particular embodiment, the biostimulator 100 can use two or more electrodes located on or within a housing of the biostimulator 100 for pacing the cardiac chamber upon receiving a triggering signal from at least one other device within the body. In an embodiment, one or more of the fixation element 106 or the pacing element 108 is an active electrode.

Leadless pacemakers or other leadless biostimulators 100 can be delivered to or retrieved from a patient using delivery or retrieval systems. The leadless biostimulator system can include delivery or retrieval systems, which may be catheter-based systems used to carry a leadless biostimulator 100 intravenously to or from a patient anatomy. The delivery or retrieval systems may be referred to collectively as transport systems. Examples of transport systems are described below. In some implementations of transport systems, a leadless pacemaker is attached or connected to a distal end of a catheter and advanced intravenously into or out of the heart 102. The transport system can include features to engage the leadless pacemaker to allow fixation of the leadless pacemaker to tissue. For example, in implementations where the leadless pacemaker includes an active engaging mechanism, such as a helical fixation element 106, the transport system can include a docking cap or key at a distal end of the catheter, and the docking cap or key may be configured to engage the leadless pacemaker and apply torque to screw the active engaging mechanism into or out of the tissue. In other implementations, the transport system includes clips designed to match the shape of a feature on the leadless pacemaker and apply torque to screw the active engaging mechanism into or out of the tissue.

When the biostimulator 100 is delivered to and screwed into the septum 104 of the heart 102, the pacing element 108 and/or the fixation element 106 may be positioned for deep septal pacing at respective bundle branches 112 in the septum 104. For example, an active electrode of the pacing element 108 can be positioned at the left bundle branch 114 in the septum 104. Similarly, the fixation element 106 can be positioned at the right bundle branch 116 in the septum 104. Optionally, one of the elements may be at a bundle branch and the other element may not be at a bundle branch.

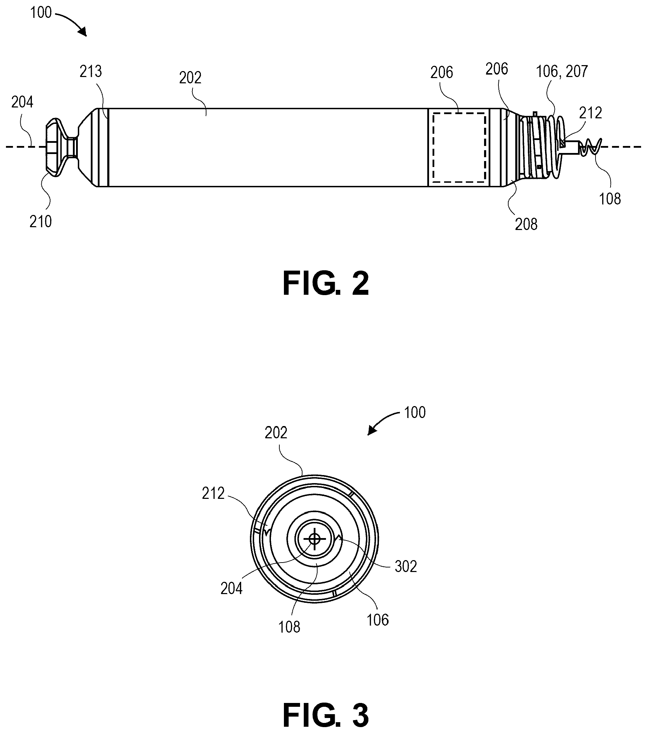

Referring to FIG. 2, a side view of a biostimulator having a pacing element is shown in accordance with an embodiment. The biostimulator 100 can be a leadless cardiac pacemaker that can perform cardiac pacing and that has many of the advantages of conventional cardiac pacemakers while extending performance, functionality, and operating characteristics. The biostimulator 100 can have two or more electrodes, e.g., a portion of the pacing element 108 that acts as an active electrode and/or a portion of the fixation element 106 that acts as an active electrode. The electrodes can deliver pacing pulses to bundle branches 112 within the septum 104 of the heart 102 to perform deep septal pacing, and optionally, can sense electrical activity from the muscle. The electrodes may also communicate bidirectionally with at least one other device within or outside the body.

In an embodiment, the biostimulator 100 includes a housing 202 having a longitudinal axis 204. The housing 202 can contain a primary battery to provide power for pacing, sensing, and communication, which may include, for example, bidirectional communication. The housing 202 can optionally contain an electronics compartment 206 (shown by hidden lines) to hold circuitry adapted for different functionality. For example, the electronics compartment 206 can contain circuits for sensing cardiac activity from the electrodes, circuits for receiving information from at least one other device via the electrodes, circuits for generating pacing pulses for delivery to tissue via the electrodes, or other circuitry. The electronics compartment 206 may contain circuits for transmitting information to at least one other device via the electrodes and can optionally contain circuits for monitoring device health. The circuit of the biostimulator 100 can control these operations in a predetermined manner. In some implementations of a cardiac pacing system, cardiac pacing is provided without a pulse generator located in the pectoral region or abdomen, without an electrode-lead separate from the pulse generator, without a communication coil or antenna, and without an additional requirement of battery power for transmitted communication.

Leadless pacemakers or other leadless biostimulators 100 can be fixed to an intracardial implant site, e.g., at the septal wall 110, by one or more actively engaging mechanisms or fixation mechanisms, such as a screw or helical member that screws into the myocardium. In an embodiment, the biostimulator 100 includes the fixation element 106 coupled to the housing 202. The fixation element 106 can include an outer helix 207 to screw into target tissue. More particularly, the biostimulator 100 can include a header assembly having a flange 208 coupled to a distal housing end 209 of the housing 202, and the fixation element 106 extending helically from the flange 208. The outer helix 207 can extend about the longitudinal axis 204 to a first distal tip 212. Accordingly, when the biostimulator 100 is delivered to the target tissue, the first distal tip 212 can pierce the tissue and the housing 202 can be rotated to screw the fixation element 106 into the target tissue to place the outer helix 207 in contact with the tissue.

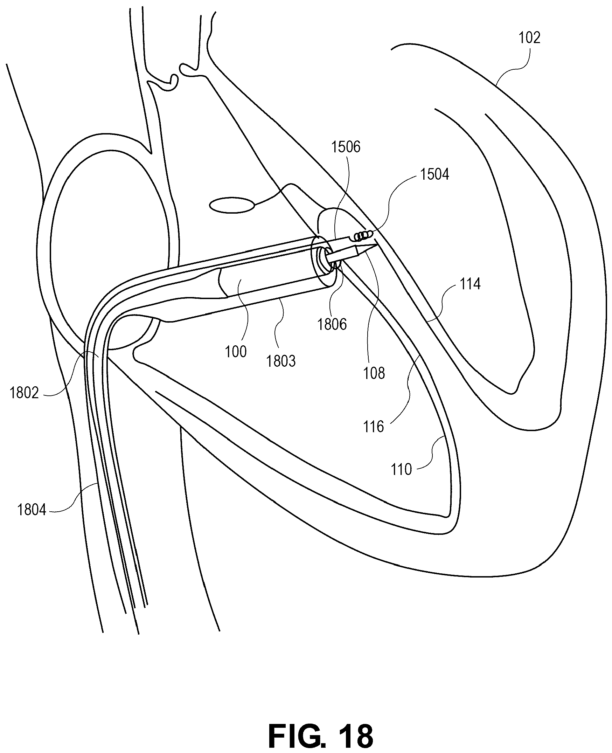

In an embodiment, the biostimulator 100 includes an attachment feature 210. The attachment feature 210 can be mounted on a proximal housing end 213 of the housing 202. More particularly, the attachment feature 210 can be mounted on an opposite end of the housing 202 from the fixation element 106 and the pacing element 108, which as described above, can be coupled to the distal housing end 209 of the housing 202. The attachment feature 210 can facilitate precise delivery or retrieval of the biostimulator 100. For example, the attachment feature 210 can be formed from a rigid material to allow a delivery or retrieval system (FIG. 18) to engage the attachment feature 210 and transmit torque to the attachment feature 210 to screw one or more of the fixation element 106 or the pacing element 108 into the target tissue.

Referring to FIG. 3, an end view of a biostimulator having a pacing element is shown in accordance with an embodiment. The biostimulator 100 can include the pacing element 108 coupled to the housing 202. The pacing element 108 can be coaxially arranged with the fixation element 106 about the longitudinal axis 204. More particularly, the pacing element 108 can extend along, e.g., axially along or helically about, the longitudinal axis 204 at a location that is radially inward from the fixation element 106. The pacing element 108 can extend distally to a second distal tip 302. The second distal tip 302 may be distal to the first distal tip 212. The pacing element 108 can be a helical element to screw into a target tissue or a conical element to pierce into the target tissue, as described below. Accordingly, when the fixation element 106 screws into the tissue, the pacing element 108 can also engage the target tissue, and the housing 202 can be advanced and/or rotated to cause the second distal tip 302 of the pacing element 108 to pierce the tissue and anchor the biostimulator 100.

One or more of the fixation element 106 or the pacing element 108 can be an active electrode, and can electrically communicate with the circuitry contained in the electronics compartment 206. Accordingly, the anchored element(s) can electrically communicate with the tissue, and can transmit electrical pulses between the tissue and the circuitry of the biostimulator 100. To facilitate electrical function of the fixation element 106 and/or the pacing element 108, the element(s) may be coated in titanium nitride to reduce a polarization value of the electrode(s). By way of example, the titanium nitride coating may be in a range of 5 to 50 microns, e.g., 13 microns.

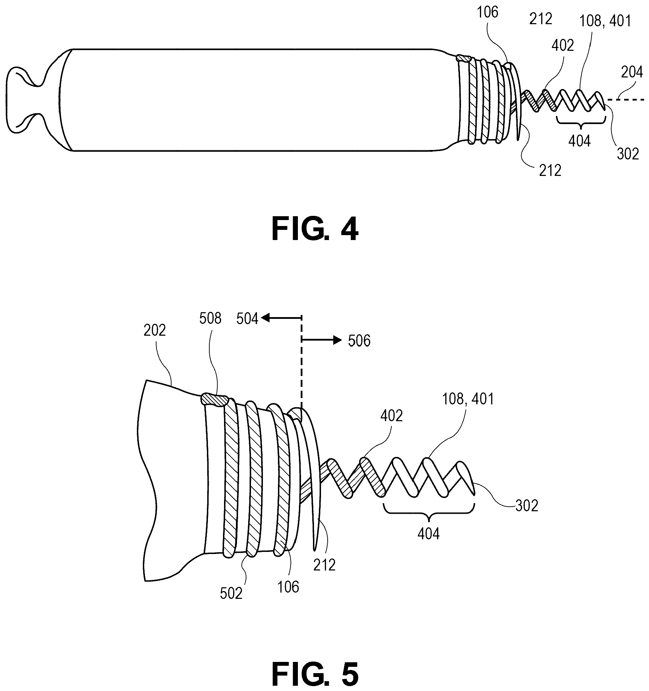

Referring to FIG. 4, a side view of a leadless biostimulator having a helical electrode is shown in accordance with an embodiment. The fixation element 106 can include a fixation helix mounted on a non-conductive helix mount, as described below. For example, the helix mount can be formed from polyether ether ketone (PEEK). The fixation element 106 can include MP35N wire having a wire diameter of 0.4-0.6 mm, e.g., 0.5 mm. The wire may be formed from a metal, such as stainless steel or a nickel cobalt alloy. The wire can be wrapped into the helix having several turns. The turns can be screwed into the septum 104 to anchor the biostimulator 100 within the heart 102.

The pacing element 108 of the biostimulator 100 can include an elongated screw. More particularly, the pacing element 108 can include a helical electrode 401 extending about the longitudinal axis 204 to the second distal tip 302. In an embodiment, the pacing element helix includes platinum-iridium wire (90-10 wire) having a diameter of 0.25-0.31 mm, e.g., 0.28 mm diameter. A major diameter of the helix can be 1.2-1.6 mm, e.g., 1.4 mm, and a length of the helix distal to the first distal tip 212 (exposed distally from the fixation element 106) may be in a range of 5-20 mm, 7 mm, 12 mm, 15 mm, or 20 mm long. Accordingly, the electrode extension can provide a means of pacing a target tissue that is distal to tissue being engaged by the fixation element 106. For example, the pacing element 108 can pace the left bundle branch 114 when the fixation element 106 is engaged near a surface of the septal wall 110 and/or to the right bundle branch 116.

In an embodiment, a pacing insulation 402 covers at least a portion of an outer surface of the pacing element 108. For example, the pacing insulation 402 can cover the outer surface between the first distal tip 212 and a distal portion 404 of the pacing element 108. The pacing insulation 402 can insulate the cathode, e.g., the helical electrode 401 of the pacing element 108, from body fluids. Coverage of the outer surface of the pacing element 108 by the insulation 402 can be tuned to optimize the pacing location. For example, the distal portion 404, e.g., the distal 1-2 turns of the helical electrode 401 of the pacing element 108 may remain exposed, and thus, the pacing element 108 can provide the cathode to target the heart tissue, e.g., the left bundle branch 114 at the second distal tip 302.

The coverage of the pacing insulation 402 over the outer surface of the pacing element 108 can also be tuned to optimize the pacing impedance. The exposed surface area of the pacing element 108 corresponds to the pacing impedance of the cathode. In an embodiment, the distal portion 404, which is uninsulated, may have a surface area that provides a pacing impedance in a range of 500-600 ohms, e.g., 550 ohms. Tuning the pacing impedance can both allow for focused stimulation of the left bundle branch 114 and improve battery longevity by requiring minimal power output of the biostimulator 100 to achieve effective deep septal pacing.

Referring to FIG. 5, a side view of a distal portion of a leadless biostimulator having a helical electrode is shown in accordance with an embodiment. The fixation element 106, like the pacing element 108, may be at least partially insulated. The fixation element 106 could be floating, meaning that the fixation element 106 may not be electrically connected to an anode or cathode of the biostimulator 100 and may be used solely for tissue attachment. In an embodiment, however, the fixation element 106 is an active electrode that provides anodic or cathodic pacing of the heart 102. Each of these embodiments is described further below, and in either case, the fixation element 106 can include an outer insulation 502 covering at least a portion of the helical fixation element 106. The outer insulation 502 may, for example, be parylene. The outer insulation 502 can cover a proximal region 504 of the fixation element 106, and a distal region 506 of the fixation element 106 is not covered by the outer insulation 502. For example, the distal region 506 can be a distal 1-2 turns, e.g., 1.5 turns, of the fixation helix. The exposed distal region 506 of the fixation element 106 can therefore provide an active electrode to pace tissue. Similar to the pacing element 108, the outer insulation 502 can be applied to tune the distal region 506 to have a pacing impedance in a range of 500-600 ohms, e.g., 550 ohms.

In an embodiment, the fixation element 106 is electrically connected to the housing 202. For example, a connection 508 can be formed between the housing 202 and the fixation element 106 by a weld, an electrical lead, etc. The housing 202 may be electrically connected to an anode of the biostimulator battery, and thus, the housing 202 can be anodic. Accordingly, the fixation element 106 may also be anodic through the connection 508 with the housing 202. When so connected, the fixation element 106 can provide anodic pacing, e.g., of the right branch bundle located superficially below a surface of the right ventricle septal wall 110, when the first distal tip 212 is engaged with the septum 104. The anodic pacing of the right branch bundle by the fixation element 106 can occur simultaneously with cathodic pacing of the left branch bundle by the pacing element 108. Simultaneous pacing of the bundle branches can improve cardiac resynchronization.

To limit anodic pacing to the region of the heart 102 in which the fixation element 106 is placed, a portion of the biostimulator proximal to the fixation element 106 may be insulated from the surrounding environment. For example, the outer insulation 502 may cover an outer surface of the attachment feature 210, the housing 202, and any other surfaces proximal to the fixation element 106. More particularly, the outer insulation 502 can cover an entire outer surface of the attachment feature 210, the housing 202, the flange 208, and any other conductive surfaces that the fixation element 106 is electrically connected to, proximal to the distal region 506.

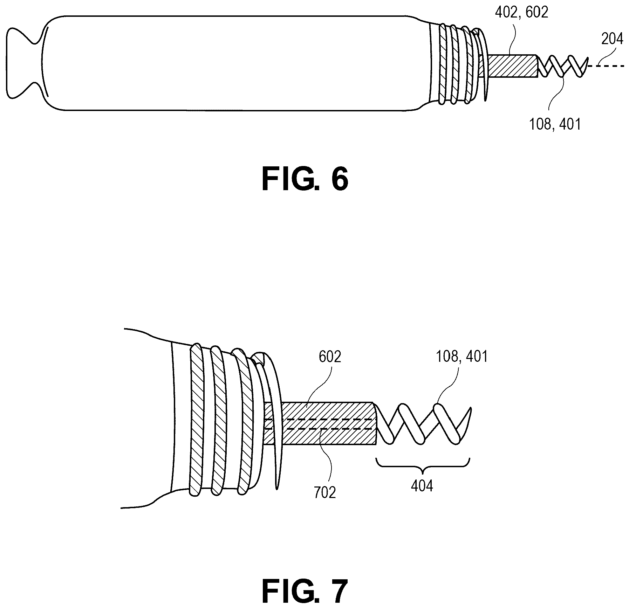

Referring to FIG. 6, a side view of a leadless biostimulator having an insulating sleeve is shown in accordance with an embodiment. The biostimulator 100 may include an insulating sleeve 602 to insulate a proximal portion of the pacing element 108. For example, the pacing insulation 402 of the biostimulator 100 may be provided by an insulating sleeve 602 that is placed over the outer surface of the pacing element 108. Accordingly, the helical electrode 401 can be exposed distal to the insulating sleeve 602.

Referring to FIG. 7, a side view of a distal portion of a leadless biostimulator having an insulating sleeve is shown in accordance with an embodiment. The insulating sleeve 602 can include a silicone rubber tube that is inserted over the proximal portion of the pacing element 108. For example, the sleeve can be loaded over the helical element of FIGS. 4-5 to replace the insulation coating, e.g., parylene, that is applied over the outer surface of the helix. Alternatively, a portion of the pacing element 108 within the insulating sleeve 602 may have a different structure than the distal portion 404 of the pacing element 108. For example, the pacing element 108 can include a conductive lead 702 extending through the insulating sleeve 602 to the distal portion 404. The conductive lead 702 can be electrically connected to the distal portion 404. The conductive lead 702 can be a straight or coiled wire or coil that is thinner and/or less stiff than the wire used to form the distal portion 404 of the pacing element 108. Furthermore, the silicone rubber section of the insulating sleeve 602 can have excellent flexibility. Accordingly, the effective bending stiffness of the proximal portion of the pacing element 108 can be lower than an effective bending stiffness of the distal portion 404 of the pacing element 108. The proximal portion can therefore limit forces applied to the tissue and reduce tissue response and scarring. This can result in improved stimulation threshold.

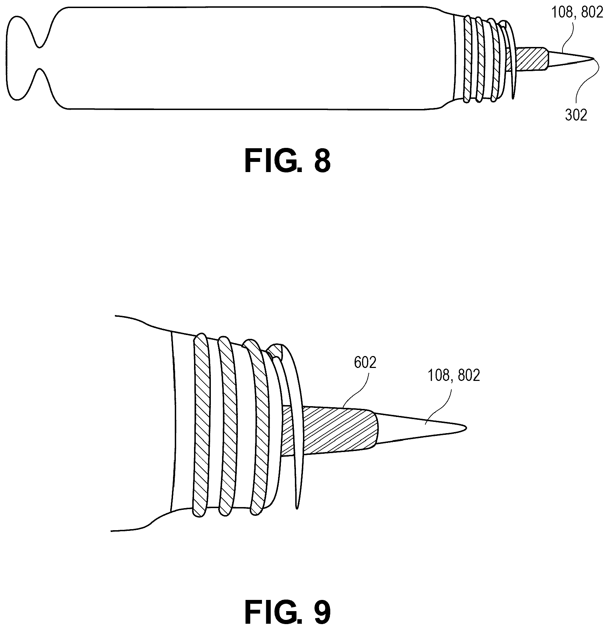

Referring to FIG. 8, a side view of a leadless biostimulator having a conical electrode is shown in accordance with an embodiment. The pacing element 108 of the biostimulator 100 may incorporate a spike-shaped electrode. More particularly, the distal portion of the pacing element 108 can include a conical electrode 802. The conical electrode 802 can extend along the longitudinal axis 204 to the second distal tip 302. Accordingly, the second distal tip 302 can have a pointed conical shape that can easily pierce heart tissue. More particularly, the spike can allow the electrode to readily penetrate the septum 104 without the need to screw in the cathode, as in the case of the helical electrode 401.

Referring to FIG. 9, a side view of a distal portion of a leadless biostimulator having a conical electrode is shown in accordance with an embodiment. The pacing element 108 can be insulated proximal to the cone-shaped end. The conical electrode 802, like the distal portion 404 of the helical electrode 401, can be exposed distal to a pacing insulation 402. The pacing insulation 402 can be coated on the pacing element 108, can be a sleeve 602 loaded over the pacing element 108 or a lead wire, etc. In any case, the pacing insulation 402 can maintain a reasonable pacing impedance of about 500 ohms at the conical electrode 802.

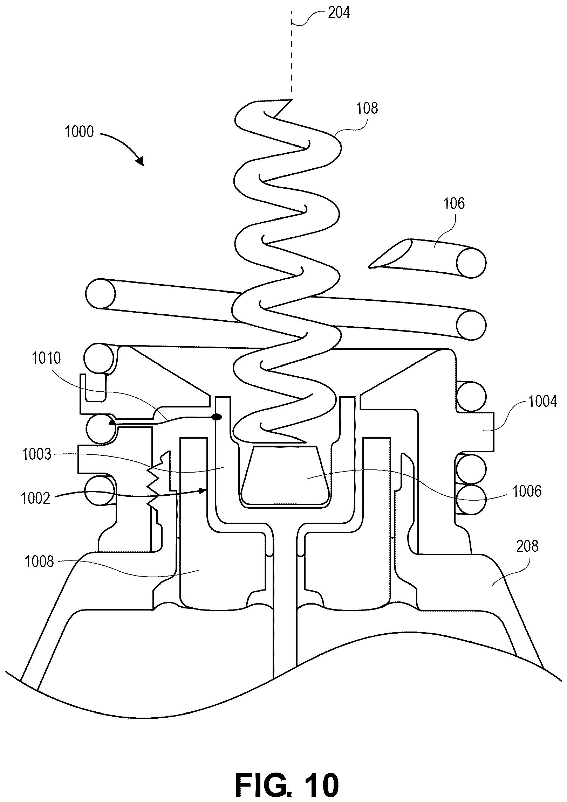

Referring to FIG. 10, a cross-sectional view of a distal portion of a biostimulator having a pacing element is shown in accordance with an embodiment. In an embodiment, the biostimulator 100 includes a header assembly 1000. The header assembly 1000 can be mounted on the housing 202, e.g., at the distal housing end 209, and can include both the fixation element 106 and the pacing element 108. As described above, the pacing element 108 can pace the left bundle branch 114 while the right bundle branch 116 is anodically paced by the fixation element 106. The fixation element 106 can be electrically connected to the housing 202, which is insulated by the outer insulation 502. Alternatively, as described below, the fixation element 106 can be electrically connected to circuitry within the housing 202 through an electrical feedthrough 1002 of the header assembly 1000. More particularly, the electrical feedthrough 1002 can be electrically connected to circuitry within the electronics compartment 206, and the fixation element 106 can be electrically connected to the electrical feedthrough 1002. Accordingly, the circuitry can be controlled to polarize the fixation element 106 to provide either anodic or cathodic pacing of the target tissue.

In an embodiment, the header assembly 1000 includes a helix mount 1004, which can be mounted on the housing 202. For example, the helix mount 1004 can have an internal thread that mounts on an external thread of the flange 208 by a threaded connection (not shown). The fixation element 106 may then be mounted on the helix mount 1004, e.g., by screwing the helical structure onto external threads of the helix mount 1004.

The electrical feedthrough 1002 of the header assembly 1000 can include an electrode cup 1003 having an electrode wall extending distally from an electrode base. The electrode wall can extend around an electrode cavity to surround a filler 1006 and separate the filler 1006 from an insulator 1008 or the flange 208 that is radially between the electrode cup 1003 of the feedthrough 1002 and the helix mount 1004. As described below, the filler 1006 can contain a therapeutic agent. The electrode cavity can be located on the longitudinal axis 204. A pin of the cup can extend proximally from the electrode base along the longitudinal axis 204 through the insulator 1008. The pin can receive pacing impulses from circuitry within the electronics compartment 206 and transmit the electrical signals to the electrode cup 1003. The impulse may then transmit into the pacing element 108, which can be coupled to, e.g., mounted within and in physical and electrical contact with, the electrode cup 1003. The pacing element 108 may then deliver the pacing impulses to the target tissue, e.g., the left bundle branch 114.

In an embodiment, the electrical feedthrough 1002 can also deliver an impulse to the fixation element 106. The fixation element 106 may be electrically connected to the electrical feedthrough 1002, e.g., the electrode cup 1003, by an interconnect lead, via, or cable extending through the helix mount 1004. The interconnect 1010 can be welded to the electrical cup and the fixation element 106, and may be conductive to transfer a pacing impulse radially outward through the helix mount 1004 to the fixation element 106. Accordingly, like the pacing element 108, the fixation element 106 may deliver pacing impulses to the target tissue, e.g., the right bundle branch 116.

It is contemplated that, although the fixation element 106 and the pacing element 108 may deliver a same, anodic or cathodic, impulse to the target tissue, a polarity of the pacing may be switched or varied between the elements. For example, a coaxial feedthrough may be used to allow a first impulse to be delivered to the pacing element 108 and a second impulse to be delivered to the fixation element 106. Polarities of the impulses may then be individually controlled to allow for any combination of anodic and cathodic pacing. For example, the left bundle branch 114 may be cathodically paced or anodically paced by the pacing element 108, and simultaneously, the right bundle branch 116 may be cathodically or anodically paced by the fixation element 106.

In an embodiment, the biostimulator 100, and more particularly the pacing element 108 is coupled to a therapeutic agent. For example, the filler 1006 within the electrode cup 1003 can contain a therapeutic agent, which may be eluted into a central lumen of the pacing element 108. The filler 1006 can be referred to as a monolithic controlled release device (MCRD). The MCRD can include the therapeutic material, which may include a corticosteroid, such as dexamethasone sodium phosphate, dexamethasone acetate, etc. Furthermore, the therapeutic agent can be loaded in a silicone matrix. Accordingly, the filler 1006 can deliver a specified dose of a therapeutic agent, e.g., a corticosteroid, into the target tissue. When the target tissue is drawn into the central lumen of the pacing element 108, the therapeutic agent can be effectively delivered into the tissue after the biostimulator 100 is implanted in a patient. Accordingly, inflammation or injury of the captured tissue may be reduced.

Other modes of coupling the therapeutic agent to the pacing element 108 may be used. For example, the filler 1006 may be a plug that is loaded directly into the central lumen of the pacing element 108. Furthermore, the filler 1006 may be a coating that is applied directly to an outer surface of the helical or conical electrode 802 described above. In an embodiment, the filler 1006 is annularly shaped, and the annular filler 1006 is loaded over an external surface of the pacing element 108 as a sleeve.

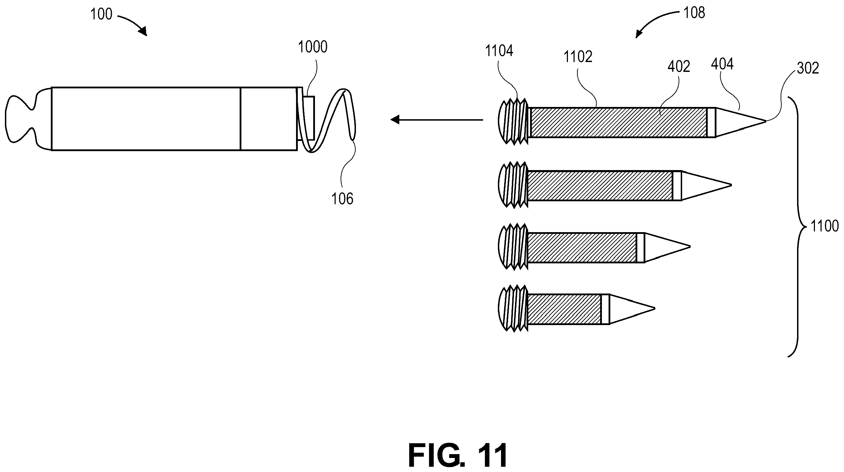

Referring to FIG. 11, a pictorial view of a biostimulator configured to receive one or more interchangeable pacing elements is shown in accordance with an embodiment. The biostimulator 100 may allow for pacing elements 108 to be interchanged. More particularly, a pacing element 108 may be selected from a group 1100 of pacing elements, e.g., based on a length of the electrode, and the selected electrode may be installed into the header assembly 1000 of the biostimulator 100. The length of the electrode may be selected based on different left branch bundle depths relative to the septal wall 110 of the right ventricle, for example.

In an embodiment, the header assembly 1000 of the biostimulator 100 includes a socket to receive a proximal end of the pacing element 108. For example, the electrode cup 1003 may have an internal thread to receive a threaded portion of the pacing element 108. More particularly, the internal thread can engage with threaded portions of any of several pacing elements 108 in a group 1100 of pacing elements having different lengths. FIG. 11 illustrates pacing element 108 lengths of 7, 10, 13, and 16 mm, respectively, for the pacing elements 108 of the group 1100, however, it will be appreciated that such lengths are provided by way of examples and other pacing element 108 lengths may be used.

Each pacing element 108 of the group 1100 can include a body 1102 extending from a proximal threaded portion 1104 to the distal portion 404. As described above, the distal portion 404 can have the distal tip of the pacing element 108. Accordingly, the proximal threaded portion 1104 can engage the biostimulator 100, and the distal portion 404 can engage the target tissue. The pacing insulation 402 on the outer surface of the body 1102 can extend between the proximal threaded portion 1104 and the distal portion 404. More particularly, the distal portion 404 can remain exposed, and the pacing insulation 402 can insulate the proximal portion of the pacing element 108, as described above. Accordingly, an impulse can be delivered through the header assembly 1000 to the proximal threaded portion 1104, and distally through the body 1102 to the distal portion 404 for pacing the target tissue.

In practice, a user can select an appropriate pacing element 108 from the group 1100 based on diagnostic data, e.g., MRI, CT scan, or echocardiogram images, that estimate a thickness of the septum 104. Alternatively, a pace mapping procedure, as described below, may be used to determine a distance between the septal wall 110 of the right ventricle and a target location, e.g., the left bundle branch 114. After identifying the distance 1711, the appropriately-sized electrode can be selected and installed in the electrode cup 1003 of the header assembly 1000. Mating of the header assembly 1000 to the pacing element 108 may be achieved using a threaded connection, however, it will be appreciated that other connection types may be used. For example, a proximal portion of the pacing element 108 may have a bayonet-type connection to engage a corresponding slot of the electrode cup 1003. Accordingly, the threaded connection is provided by way of example and not limitation.

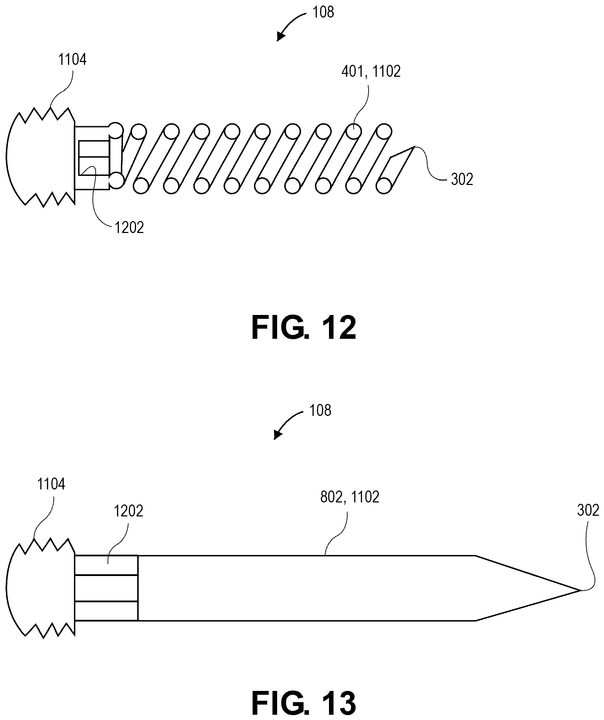

Referring to FIG. 12, a side view of a pacing element having an internal torque feature is shown in accordance with an embodiment. The pacing element 108 can include the helical electrode 401, as described above. In an embodiment, the pacing element 108 includes a torque feature 1202 to receive a head of a tool (not shown). The tool can be used to torque the body 1102, and thus, screw the proximal threaded portion 1104 into the threaded hole of the biostimulator 100.

In an embodiment, the torque feature 1202 can be an internal feature. More particularly, the torque feature 1202 can be internal to the body 1102. For example, the internal feature may be a hex socket to receive a hex head of a tool. The tool may, for example, be a torque-limited wrench. The wrench may therefore be used to engage the internal torque feature 1202 and screw the pacing element 108 into the header assembly 1000 with an appropriate assembly torque.

Referring to FIG. 13, a side view of a pacing element having an external torque feature is shown in accordance with an embodiment. The pacing element 108 can include the conical electrode 802, as described above. In an embodiment, the torque feature 1202 that receives the tool head is external to the body 1102. For example, the external feature can be a surface indentation, a hexagonal surface shape, etc., that will engage the tool head to allow the tool to grip and/or torque the pacing electrode. Accordingly, the tool can assemble the pacing element 108 to the header assembly 1000 of the biostimulator 100.

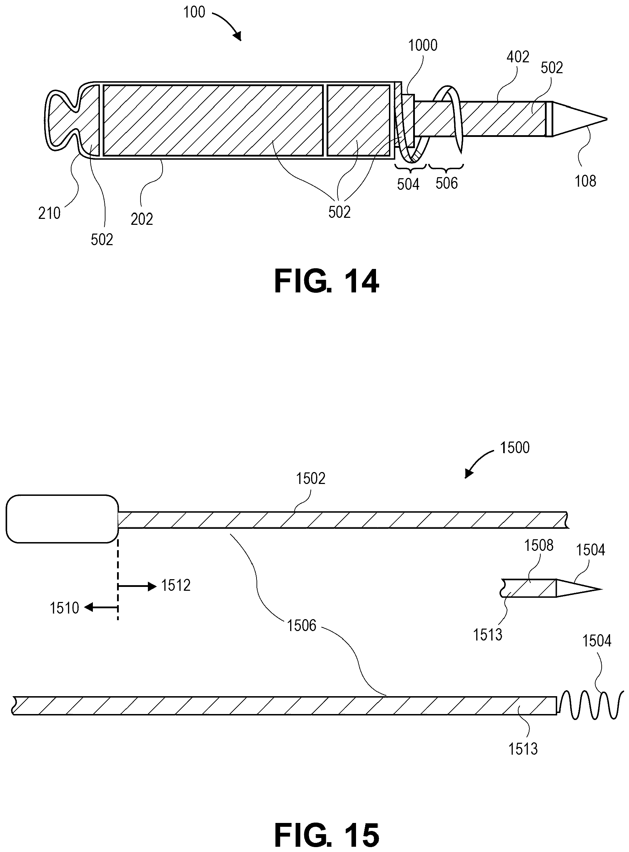

Referring to FIG. 14, a side view of a leadless biostimulator having an outer insulation is shown in accordance with an embodiment. The side view illustrates that, in an embodiment, the outer insulation 502 covering an external surface of the biostimulator 100 can extend over an entirety of the attachment feature 210, the housing 202, and the header assembly 1000. The insulation, which may be a parylene coating, can therefore insulate virtually all of the external surface of the biostimulator 100. The insulation may also cover the proximal region 504 of the fixation element 106, and leave the distal region 506 of the fixation element 106 exposed, as described above. Accordingly, the biostimulator 100 can deliver pacing impulses through the exposed regions of the pacing element 108 and the fixation element 106 to perform deep septal pacing.

Referring to FIG. 15, a side view of a pace mapping system is shown in accordance with an embodiment. A pace mapping system 1500 can be used to pace map and fix the biostimulator 100 to tissue in or near the left bundle branch 114. More particularly, as described below, the pace mapping system 1500 can be used to evaluate a depth of penetration for the pacing element 108, and can include an element that may be left in place to provide a landmark as the user guides the biostimulator 100 to the implantation site. In an embodiment, the placement sheath of the pace mapping system 1500 may be used as a guidewire, e.g., an "over the wire" guidewire for delivering the biostimulator 100 to the implantation site.

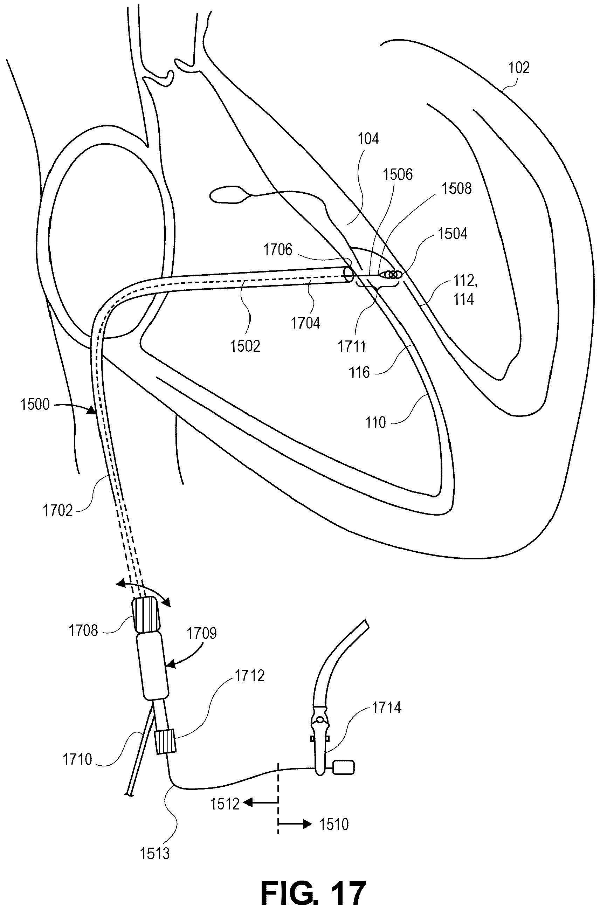

The pace mapping system 1500 can include a pace mapping tool 1502 designed to be placed into a steerable sheath (FIG. 17). The pace mapping tool 1502 may first be used to identify the His potential and the His location using a sensing tip 1504. More particularly, the pace mapping tool 1502 can include an elongated body 1506 having a distal body end 1508, and the sensing tip 1504 can be coupled to the distal body end 1508. The sensing tip 1504 may be configured to pierce a target tissue, e.g., the septum 104, to engage and monitor the potential of the target tissue. For example, the sensing tip 1504 can have a helical or conical configuration, similar to the distal tip of the pacing element 108, to facilitate tissue puncture. The mapping tool may employ an alternative sensing tip 1504 as shown in FIG. 15. More particularly, the sensing tip 1504 can be sharp and conical. If a diameter of the elongated body 1506 and/or sensing tip 1504 is small enough, the electrode at the sensing tip 1504 can be beveled or blunt. Owing to its small diameter, the mapping electrode can easily penetrate tissue.

The sensing tip 1504 can be electrically connected to a proximal body portion 1510 through the elongated body 1506. For example, the proximal body portion 1510 can include a tab, plug, or other electrical connector that is electrically connected to the sensing tip 1504 through an electrical lead extending through the elongated body 1506. Accordingly, an electrogram (EGM) monitoring system or a pacing system analyzer (PSA) programmer can connect to the proximal body portion 1510 outside of the patient anatomy to monitor the sensing tip 1504 when it has pierced the target tissue within the patient anatomy.

In an embodiment, the elongated body 1506 includes a distal body portion 1512 extending longitudinally from the proximal body portion 1510 to the distal body end 1508. A body insulation 1513, such as a parylene coating, can cover the distal body portion 1512. The body insulation 1513 may alternatively include a thin polymeric coating, such as a PTFE, ETFE, or polyimide sheath or coating. The proximal body portion 1510 may, however, remain exposed (may not be covered by the body insulation 1513). Accordingly, the proximal body portion 1510 can connect to a monitoring system to sense tissue potential through the sensing tip 1504.

A method comprises delivering a pace mapping system to a septal wall of a heart, wherein the pace mapping system includes a steerable sheath having a central lumen extending to a distal sheath end. The method also comprises advancing a pace mapping tool of the pace mapping system through the central lumen and the septal wall to a bundle branch in the septal wall, wherein the pace mapping tool includes an elongated body extending through the central lumen to a distal body end, and a sensing tip coupled to the distal body end. The method further comprises determining a distance between the distal sheath end at the septal wall and the sensing tip at the bundle branch. The method additionally comprises delivering a biostimulator to the septal wall, wherein the biostimulator includes a pacing element selected based on the distance.

Delivering the biostimulator preferably includes delivering a transport system over the pace mapping tool to the septal wall, wherein the transport system includes a catheter having a distal catheter end, and wherein the biostimulator is coupled to the distal catheter end.

The method optionally comprises selecting the pacing element based on the distance and installing the pacing element into the biostimulator.

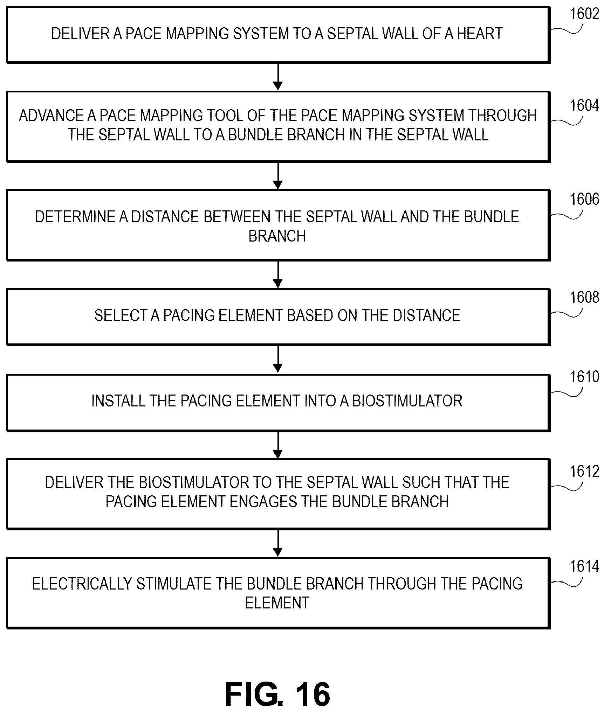

Referring to FIG. 16, a flowchart of a method of stimulating a bundle branch using a biostimulator having a pacing element is shown in accordance with an embodiment. Some operations of the method are shown in FIGS. 17-18. Accordingly, FIGS. 16-18 are described together in the following description.

At operation 1602, the pace mapping system 1500 is delivered to the septal wall 110 of the heart 102. Referring to FIG. 17, in an embodiment, the pace mapping system includes a steerable sheath 1702 having a central lumen 1704 extending to a distal sheath end 1706. The steerable sheath 1702 can be placed in the femoral vein in the area of the groin (not shown). The steerable sheath 1702 is long enough to run from the groin to the right ventricle septum 104, e.g., about 100 to 130 cm. The steerable sheath 1702 includes a steerable hollow catheter having the central lumen.

The steerable sheath 1702 can include a steering feature 1708 to allow a user to steer the distal sheath end 1706 to the target implantation site. For example, the pace mapping system 1500 can include a handle 1709 coupled to the steerable sheath 1702, and the handle 1709 can include the steering feature 1708. The steering feature 1708 can be a knob, a lever, or another mechanism that the user can manipulate at a proximal end of the sheath to cause the sheath to bend or curve such that the distal sheath and is steered to the target site.

The steerable sheath 1702 can be connected to a flush tube 1710, which can be connected to a stopcock (not shown) and used to receive a heparin flush from an attached syringe that flushes the steerable sheath 1702. A knob 1712 can be used to close a hemostasis valve around the pace mapping tool 1502. Accordingly, the pace mapping tool 1502 can be slidably disposed within the steerable sheath 1702, and can be reversibly sealed and attached thereto. In an embodiment, an alligator clip 1714 is in electrical contact with the pace mapping tool 1502 and allows for sensing and pacing from the sensing tip 1504.

It is not necessary to screw the sensing tip 1504 of the pace mapping tool 1502 into the His region. The distal end of the mapping tool can be retracted into the steerable sheath 1702 during sheath delivery. The steerable sheath 1702 can be steered to the septal site in the right ventricle, approximately 1.5 cm below the His location. This location allows the pace mapping tool 1502 to be advanced out of the steerable sheath 1702 to sense locations for deep septal pacing.

At operation 1604, the pace mapping tool 1502 is advanced through the septal wall 110 to a bundle branch 112 in the septal wall 110. More particularly, the pace mapping tool 1502 can be advanced through the central lumen of the steerable sheath 1702 until the sensing tip 1504 engages the septal wall 110. The helical or conical tip of the sensing tip 1504 can be screwed or pushed into the septal tissue by rotating or pushing the knob 1712 to advance the elongated body 1506 of the pace mapping tool 1502. The knob 1712 can be further turned or pushed to advance the sensing tip 1504 into the tissue while pace mapping and observing a 12 lead electrocardiogram (EKG) looking for a short pace to R-wave interval of about 80 milliseconds to V4-V6 with a right bundle branch block (RBBB) morphology in the paced EKG indicating that the screw is near or in the left bundle branch 114. A threshold can be in the range of 0.5 V at 0.5 ms when the left bundle branch 114 is paced.

At operation 1606, a distance 1711 between the distal sheath end 1706 at the septal wall 110 and the sensing tip 1504 at the bundle branch 112 can be determined. Referring to FIG. 17, the depth of the sensing tip 1504 is measured from the sensing tip 1504 to the distal sheath end 1706 of the steerable sheath 1702, which is placed against the right ventricle septal wall 110. This measurement can be used to select an appropriately sized (suitable length) pacing element 108 from the group 1100 of pacing elements 108.

At operation 1608, the pacing element 108 is selected based on the distance 1711. The pacing element 108 having a body length that is equal to the depth of the sensing tip 1504 can be selected. Accordingly, the tip of the pacing element 108 can engage the left bundle branch 114 when the fixation element 106 is screwed into the septal wall 110.

At operation 1610, the pacing element 108 is installed into the biostimulator 100. The pacing element 108 may be inserted and locked to the header assembly 1000. For example, a torque limiting device can be used to grip and screw the pacing element 108 into the header assembly 1000, as described above.

Referring to FIG. 18, at operation 1612, the biostimulator 100 is delivered to the septal wall 110. The biostimulator 100 includes the pacing element 108 selected based on the distance 1711. In an embodiment, the biostimulator 100 can be delivered by a transport system 1802. In an embodiment, the transport system 1802 is a delivery system or a retrieval system that is delivered to the implantation site over the pace mapping tool 1502. For example, the transport system 1802 can include a catheter 1804 having a distal catheter end 1806 that can be steered to the implantation site. In an embodiment, the biostimulator 100 is coupled to the distal catheter end 1806. For example, the biostimulator 100 can be contained within a protective sheath 1803 of the transport system 1802 at the distal catheter end 1806 and/or the attachment feature 210 of the biostimulator 100 can be retained by a docking cap of the catheter 1804. The protective sheath 1803 can be long enough to house the biostimulator 100. Accordingly, when the transport system 1802 is delivered to the implantation site, then the biostimulator 100 may also be delivered to the septal wall 110.

The transport system 1802 may be delivered to the implantation site over the elongated body 1506 of the pace mapping tool 1502. The pace mapping tool 1502 can remain engaged to the target tissue, and the knob 1712 can be rotated to unlock the elongated body 1506 of the pace mapping tool 1502 from the steerable sheath 1702. The steerable sheath 1702 may then be removed from the venous system over the elongated body 1506, leaving the pace mapping tool 1502 in place.

With the sensing tip 1504 engaged to the pacing site, e.g., the left bundle branch 114, the transport system 1802 may be loaded onto the elongated body 1506 and advanced distally. In an embodiment, the catheter 1804 includes a lumen to receive the elongated body 1506, e.g., in an over-the-wire fashion. Alternatively, a lumen within the catheter 1804 that is coaxial with the docking cap may be sized to receive the proximal potion of the elongated body 1506. In either case, the elongated body 1506 and/or sensing tip 1504 can provide visibility and/or act as a landmark to help steer the transport system 1802 to the appropriate pacing site. Optionally, the elongated body 1506 can have sufficient stiffness to act as a guidewire to support the transport system 1802 when tracking the transport system 1802 distally through the vasculature to the septal wall 110.

The electrode of the biostimulator 100 can be inserted at a site immediately adjacent to the elongated body 1506 of the pace mapping tool 1502. The left bundle branch 114 is relatively diffuse, and therefore, a carefully delivered biostimulator 100 will electrically engage the left bundle branch 114 as marked by the sensing tip 1504. After verifying capture of the left bundle branch 114, the sensing tip 1504 may be removed, e.g., unscrewed, and pulled back slightly. The protective sheath 1803 of the transport system 1802 may be pulled back and the biostimulator 100 may be undocked from the steerable catheter 1804. The transport system 1802 and the pace mapping tool 1502 may be removed with the tip of the pace mapping tool 1502 in the protective sheath 1803, or even fully retracted from the transport system 1802 before removing the transport system 1802.

At operation 1614, the bundle branch 112 is electrically stimulated through the pacing element 108. When the biostimulator 100 was advanced into the target tissue, the fixation element 106 can anchor within the septal wall 110, e.g., at the right bundle branch 116, and the pacing element 108 can extend to the left bundle branch 114. Electrical impulses may then be delivered through one or both of the elements to pace the bundle branch(es). As described above, there is the option of using the fixation element 106 to perform anodic and/or cathodic pacing of the right bundle branch 116. Accordingly, the biostimulator 100 can activate the left and/or right portions of the heart 102 synchronously to perform deep septal pacing.

In the foregoing specification, the invention has been described with reference to specific exemplary embodiments thereof. It will be evident that various modifications may be made thereto without departing from the scope of the invention as set forth in the following claims. The specification and drawings are, accordingly, to be regarded in an illustrative sense rather than a restrictive sense.

## Claims

1. A biostimulator (100), comprising:
a housing (202) having a longitudinal axis (204) and containing an electronics compartment (206);
a fixation element (106) coupled to the housing (202), wherein the fixation element (106) includes an outer helix (207) extending about the longitudinal axis (204) to a first distal tip (212); and
a pacing element (108) coupled to the housing (202), wherein the pacing element (108) extends along the longitudinal axis (204) to a second distal tip (302) distal to the first distal tip (212), and wherein a pacing insulation (402) covers an outer surface of the pacing element (108) between the first distal tip (212) and a distal portion (404) of the pacing element (108) having the second distal tip (302).

2. The biostimulator of claim 1, wherein the distal portion (404) of the pacing element (108) includes a helical electrode (401) extending about the longitudinal axis (204) to the second distal tip (302).

3. The biostimulator of claim 1 or 2, wherein the pacing insulation (402) includes an insulating sleeve (602).

4. The biostimulator of claim 3, wherein the pacing element (108) includes a conductive lead (702) extending through the insulating sleeve (602) to the distal portion (404).

5. The biostimulator of any one of claims 1 to 4, wherein the distal portion (404) of the pacing element (108) includes a conical electrode (802) extending along the longitudinal axis (204) to the second distal tip (302).

6. The biostimulator of any one of claims 1 to 5, further comprising:
an attachment feature (210) mounted on a proximal housing end (213) of the housing (202), wherein the fixation element (106) and the pacing element (108) are coupled to a distal housing end (209) of the housing (202); and
an outer insulation (502) covering the attachment feature (210), the housing (202), and a proximal region (504) of the fixation element (106), wherein a distal region (506) of the fixation element (106) is not covered by the outer insulation (502).

7. The biostimulator of any one of claims 1 to 6, wherein the distal portion (404) of the pacing element (108) has a pacing impedance in a range of 450 to 650 ohms.

8. The biostimulator of any one of claims 1 to 7, wherein the fixation element (106) is electrically connected to the housing (202).

9. The biostimulator of any one of claims 1 to 8, further comprising a header assembly (1000) mounted on the housing (202), wherein the header assembly (1000) includes an electrical feedthrough (1002) electrically connected to circuitry within the electronics compartment (206), and wherein the fixation element (106) is electrically connected to the electrical feedthrough (1002).

10. The biostimulator of any one of claims 1 to 9, further comprising a therapeutic agent coupled to the pacing element (108).

11. A pacing element (108) for a biostimulator (100), comprising:
a body (1102) extending from a proximal threaded portion (1104) to a distal portion (404) having a distal tip (302); and
a pacing insulation (402) on an outer surface of the body (1102) between the proximal threaded portion (1104) and the distal portion (404).

12. The pacing element of claim 11 further comprising a torque feature (1202) to receive a tool head to screw the proximal threaded portion (1104) into a threaded hole of the biostimulator (100).

13. The pacing element of claim 12, wherein the torque feature (1202) is internal to the body (1102).

14. The pacing element of claim 12, wherein the torque feature is external to the body (1102).

15. A pace mapping system (1500), comprising:
a steerable sheath (1702) having a central lumen (1704); and
a pace mapping tool (1502) including an elongated body (1506) having a distal body end (1508), and a sensing tip (1504) coupled to the distal body end (1508) and configured to pierce a target tissue (104).

16. The pace mapping system of claim 15, wherein the elongated body (1506) includes a distal body portion (1512) extending longitudinally from a proximal body portion (1510) to the distal body end (1508), and wherein a body insulation (1513) covers the distal body portion (1508) and not the proximal body portion (1510).

17. The pace mapping system of claim 15 or 16, further comprising a handle (1709) coupled to the steerable sheath (1702), wherein the handle (1709) includes a steering feature (1708) configured to steer a distal sheath end (1706) of the steerable sheath (1702).
